# EUROPÄISCHE PATENTANMELDUNG

(11) **EP 4 371 761 A1**
(43) Veröffentlichungstag der Anmeldung: **22.05.2024**
(21) Anmeldenummer: 23202471.1
(22) Anmeldetag: 09.10.2023
(51) Int. Cl.: B32B 5/26, A47L 13/17, B32B 5/06, B32B 5/08

(54) **UMWELTVERTRÄGLICHES IMPRÄGNIERTES EINWEG-TUCHPRODUKT**

(30) Priorität: 18.11.2022 DE 102022130589
(71) Anmelder: Dr. Schumacher GmbH, 34323 Malsfeld-Beiseförth (DE)
(72) Erfinder: Pogrzeba, Jessica, 34323 Malsfeld (DE); Rehm, Marie-Elisabeth, 34323 Malsfeld (DE); Pohl, Kai-Uwe, 34323 Malsfeld (DE); Schweinsberg, Selina, 34323 Malsfeld (DE); Ballez, Mike, 34323 Malsfeld (DE); Birkelbach, Anke, 34323 Malsfeld (DE)
(74) Vertreter: Sandvoß, Stefanie

(57) **Zusammenfassung**

Die Erfindung betrifft ein mehrlagiges imprägniertes Einweg-Tuchprodukt für die Körperreinigung und/oder -pflege und/oder für die Reinigung und/oder Pflege von medizinischen Sonden, elektronischen Geräten, Fenstern, Möbeln und/oder Fahrzeugen, wobei das Einweg-Tuchprodukt mindestens eine Reinigungsschicht und eine Kernschicht umfasst, wobei sowohl die Reinigungsschicht als auch die Kernschicht cellulose-basierte Fasern enthalten und vollständig aus Naturfasern und/oder Regeneratfasern und/oder einem oder mehreren Biopolymeren bestehen.

## Beschreibung

Die vorliegende Erfindung betrifft ein umweltverträgliches imprägniertes Einweg-Tuchprodukt sowie ein Verfahren zu dessen Herstellung.

Feuchttücher sind bekanntermaßen Vliesstoffe, die mit einer in der Regel flüssigen Zubereitung getränkt sind, die u.a. einen reinigenden und/oder pflegenden Inhaltsstoff enthält. Diese mit einem Tränkungsmittel getränkten Tücher finden mittlerweile in vielen Bereichen der persönlichen Pflege und vor allem Babypflege Anwendung und erfreuen sich sehr hoher Beliebtheit, da sie die Möglichkeit einer schnellen und bequemen Reinigung bzw. Pflege bieten, ohne dass dafür zusätzliches Wasser erforderlich ist. In der Regel zu handlichen Einheiten von ca. 10 bis 100 Tüchern verpackt, zeichnen sich Feuchttücher darüber hinaus auch durch ihre Portabilität aus und sind vor allem auch aufgrund dieser Eigenschaft aus unserer immer mobiler werdenden Gesellschaft nicht mehr wegzudenken.

Gegenüber einer herkömmlichen Körperreinigung mit wiederverwendbaren und waschbaren Stoffen, auf die Körperreinigungsmittel aufgetragen werden, wie z.B. Waschlappen oder Waschhandschuhe, besteht bei der Verwendung von mit einem Tränkungsmittel imprägnierten Einweg-Tuchprodukten der Vorteil, dass diese nach der Benutzung entsorgt werden. Dieser Vorteil existiert auch bei der Verwendung von imprägnierten Einweg-Tuchprodukten für die Reinigung von Flächen, die anstelle von wiederverwendbaren und waschbaren Stoffen z.B. für die Reinigung von Sanitärobjekten und Böden eingesetzt werden können.

Denn bei Verwendung von wiederverwendbaren und waschbaren Stoffen für die Reinigung ist es möglich, dass vergessen wird, diese gründlich zu waschen und sie stattdessen nur unter fließendem Wasser ausgespült werden, wobei Keime auf der Oberfläche verbleiben und sich verbreiten können. Auch bei einem nicht sofortigen Waschen verbreiten sich die auf der Oberfläche eines wiederverwendbaren Tuchprodukts vorkommenden Keime, wenn dieses z.B. bis zum nächsten Starten eines Waschgangs in der Waschmaschine zwischengelagert wird. Auf diese Weise stellt das eigentlich zur Reinigung bestimmte Tuchprodukt sogar ein Risiko für verschleppte Kontaminationen bzw. eine Quelle von Kontaminationen dar.

Diesem Problem wird im Stand der Technik durch Bereitstellung von Einweg-Tuchprodukten begegnet, die beispielsweise zur Reinigung bzw. Desinfektion von Flächen vorgesehen sind und mit einem antimikrobiell wirkenden Tränkungsmittel vorgetränkt sein können, oder die zur Körperreinigung von z.B. Babys und Kleinkindern mit einem geeigneten und hautfreundlichen Tränkungsmittel imprägniert sein können.

Es ist bekannt, dass entsprechende vorgetränkte Einweg-Tuchprodukte mehrere Schichten Vliesstoff umfassen können, die in der Regel unterschiedlich zusammengesetzt sind. Dies bewirkt einerseits eine hinreichende Stabilität des Tuchprodukts und kann andererseits bei Schichten aus unterschiedlich zusammengesetzten Vliesstoffen bei entsprechender Wahl der Vliesstoffe gewährleisten, dass beispielsweise eine erste Schicht eine gute Flüssigkeitsaufnahme bietet, während eine zweite Schicht eine gute Flüssigkeitsabgabe bietet.

Die WO 02/36339 A2 beschreibt beispielsweise ein solches mehrschichtiges vorgetränktes Wischtuch, dessen mindestens zwei Schichten eine Mischung aus hydrophilen und hydrophoben Fasern enthalten. Das Wischtuch umfasst dabei zumindest eine Reinigungsschicht sowie eine Schicht, die als Flüssigkeitsreservoir dient.

Ein ähnlicher Ansatz für ein mehrschichtiges Einweg-Bodenwischtuch wird in der EP 1 212 478 A1 verfolgt. Eine antimikrobielle Eigenschaft wird den Bodenwischtüchern durch das Hinzufügen von vorzugsweise Silberpartikeln verliehen.

Die US 4298649 A beschreibt ein mehrschichtiges Bodenwischtuch aus mehreren Schichten Polypropylen mit unterschiedlicher Erscheinung, wobei das Bodenwischtuch Tenside enthält. An dieser Lösung ist nachteilhaft, dass das Bodenwischtuch aufgrund des hohen Kunststoffgehaltes als Einweg-Artikel nicht umweltfreundlich ist.

Auch für die Körperreinigung und -pflege bestimmte herkömmliche Feuchttücher mit mehrschichtigem Aufbau weisen einen Kunststoffgehalt auf, der ein Verschweißen der einzelnen Schichten miteinander erlaubt. Dem eingangs genannten Vorteil des Vermeidens von Kontaminationen bei der Verwendung von Einweg-Feuchttüchern steht hier allerdings der Nachteil einer hohen Belastung für die Umwelt gegenüber, der daraus resultiert, dass die Einweg-Tuchprodukte bestimmungsgemäß in großer Vielzahl weggeworfen werden und somit ein in der Summe großer Kunststoffanteil als Abfall anfällt, den es zu entsorgen gilt.

Die DE 10 2021 122 041 B1 beschreibt ein Bodenwischtuch mit abrasivem Streifen, dessen einzelne Schichten wie auch der abrasive Streifen vollständig aus Naturfasern und/oder Regeneratfasern bestehen. Aufgrund des abrasiven Streifens ist das Bodenwischtuch jedoch nicht zur Reinigung empfindlicher Oberflächen geeignet, da diese durch den abrasiven Streifen zerkratzen würden bzw. eine Anwendung auf der Haut äußerst unangenehm wäre.

### Aufgabe der Erfindung

Es ist die Aufgabe der vorliegenden Erfindung, ein alternatives mehrlagiges Einweg-Tuchprodukt für die Körperreinigung und -pflege bereitzustellen, das die vorgenannten aus dem Stand der Technik bekannten Nachteile überwindet und sich insbesondere durch eine ausgezeichnete Umweltverträglichkeit sowie durch ein vereinfachtes Herstellungsverfahren auszeichnet.

### Allgemeine Beschreibung der Erfindung

Die Erfindung löst diese Aufgabe mit den Merkmalen der Ansprüche und insbesondere mit einem mehrlagigen Einweg-Tuchprodukt, das mindestens eine Reinigungsschicht und eine Kernschicht umfasst, wobei sowohl die Reinigungsschicht als auch die Kernschicht cellulose-basierte Fasern enthalten und vollständig aus Naturfasern und/oder Regeneratfasern und/oder einem oder mehreren Biopolymer(en) bestehen.

Cellulose-basierte Fasern umfassen natürliche Cellulosefasern wie Baumwolle, Bast-Fasern wie Hanf und Flachs sowie man-made Cellulose-Fasern (Cellulose-Regeneratfasern) wie Viskose und Lyocell.

Gemäß einer Ausführungsform besteht das erfindungsgemäße Einweg-Tuchprodukt aus der Reinigungsschicht und der Kernschicht und weist keine weiteren Schichten oder sonstige Bestandteile auf.

Gemäß einer anderen Ausführungsform besteht das erfindungsgemäße Einweg-Tuchprodukt aus der Reinigungsschicht, der Kernschicht sowie einer oder mehreren weiteren Schichten, wobei die eine oder mehreren weiteren Schichten vorzugsweise auch vollständig aus Naturfasern und/oder Regeneratfasern und/oder einem oder mehreren Biopolymeren bestehen. Eine weitere Schicht kann insbesondere eine vollständig aus Naturfasern und/oder Regeneratfasern und/oder einem oder mehreren Biopolymeren bestehende Deckschicht sein, die auf der der Reinigungsschicht abgewandten Seite der Kernschicht angeordnet ist.

Gemäß einer weiteren Ausführungsform weist das erfindungsgemäße Einweg-Tuchprodukt außer der Reinigungsschicht und der Kernschicht noch eine weitere Schicht auf, die flüssigkeitsundurchlässig ist und als Sperrschicht fungiert. Diese weitere Schicht kann insbesondere auf der der Reinigungsschicht abgewandten Seite der Kernschicht angeordnet sein und bewirkt dort, dass Flüssigkeit bzw. Tränkungsmittel nicht nach oben, d.h. von der Kernschicht, sondern ausschließlich nach unten, d.h. von der Reinigungsschicht, abgegeben wird. Die weitere Schicht besteht dabei vorzugsweise aus nachwachsenden und/oder biologisch abbaubaren Rohstoffen bzw. Biopolymeren, wie beispielsweise PLA (Polymilchsäure) oder Maisstärke.

Gemäß noch einer weiteren Ausführungsform besteht das erfindungsgemäße Einweg-Tuchprodukt aus der Reinigungsschicht, der Kernschicht, optional einer oder mehreren weiteren Schichten, die vorzugsweise ebenfalls vollständig aus Naturfasern und/oder Regeneratfasern und/oder einem oder mehreren Biopolymeren bestehen, einem Tränkungsmittel, mit dem eine oder mehrere der vorgenannten Schichten vorgetränkt ist bzw. sind, sowie Befestigungsmitteln zum Befestigen des erfindungsgemäßen Einweg-Tuchproduktes an einem Applikator.

Die Befestigungsmittel umfassen dabei z.B. Bänder, Knöpfe, Haken, einen Reißverschluss und/oder einen Klettverschluss, wobei die Befestigungsmittel vorzugsweise an der optionalen Deckschicht angeordnet sind und wobei das erfindungsgemäße Einweg-Tuchprodukt mittels dieser Befestigungsmittel an einem Applikator befestigt werden kann. Alternativ kann die Deckschicht auch als Gegenschicht für die vorgenannten Befestigungsmittel dienen, die diese Befestigungsmittel nicht selbst aufweist, sondern mit diesen in Eingriff gebracht werden kann, beispielsweise indem ein an einem Applikator angeordneter Klettverschluss an der Deckschicht befestigt wird oder ein an einem Applikator angeordneter Haken oder Knopf in eine an der Deckschicht angeordnete Öse bzw. Schlaufe oder ein Loch eingreift, die ebenfalls als Befestigungsmittel verstanden werden.

Generell ist es bevorzugt, dass die optionalen Befestigungsmittel entweder aus einem kompostierbaren Material bestehen, oder sofern sie nicht aus einem kompostierbaren Material bestehen, auf einfache Weise, z.B. durch Abtrennen entlang einer Perforation, nach der erfolgten Körperreinigung oder -pflege von dem restlichen Einweg-Tuchprodukt entfernt und separat entsorgt werden können.

Erfindungsgemäß enthalten die Reinigungsschicht, die Kernschicht, die optionale Deckschicht sowie weitere optionale Schichten Naturfasern und/oder Regeneratfasern. Unter "Naturfasern" werden alle Fasern natürlichen Ursprungs verstanden, die sich ohne weitere chemische Umwandlungsreaktionen direkt einsetzen lassen. "Regeneratfasern" sind hingegen Fasern, die aus natürlich vorkommenden, nachwachsenden Rohstoffen über chemische Prozesse hergestellt werden. Wie Naturfasern bestehen jedoch auch Regeneratfasern zu 100 % aus Naturstoff.

Vorzugsweise sind die in der Reinigungsschicht, in der Kernschicht, in der optionalen Deckschicht und in optionalen weiteren Schichten des erfindungsgemäßen Einweg-Tuchprodukts enthaltenen Naturfasern und/oder Regeneratfasern unabhängig voneinander aus der Gruppe ausgewählt, die Viskosefasern, Modalfasern, Lyocellfasern, Cellulosefasern, Cuprofasern, Baumwollfasern, Jutefasern, Hanffasern und Flachsfasern sowie beliebige Mischungen dieser umfasst oder daraus besteht.

Ein in der Reinigungsschicht, in der Kernschicht oder optionalen weiteren Schichten enthaltenes bevorzugtes Biopolymer ist PLA.

Aus dem Stand der Technik bekannte mehrschichtige Tuchprodukte weisen im Gegensatz zu dem erfindungsgemäßen Tuchprodukt immer einen Kunstfaseranteil auf, beispielsweise einen PP-Anteil oder PET-Anteil, der eine thermische Verbindung der einzelnen Schichten des Tuchprodukts erlaubt. Die Verbindung der einzelnen Schichten eines erfindungsgemäßen Tuchprodukts erfolgt hingegen beispielsweise durch sog. "Mikro-Entangling" über Ultraschallbewegung. Dabei werden die in den einzelnen Schichten enthaltenen Natur- und/oder Regeneratfasern so in Bewegung gebracht, dass sie sich dabei ineinander verhaken und verfilzen. Alternativ kann die Verbindung der einzelnen Schichten eines erfindungsgemäßen Einweg-Tuchprodukts auch durch eine Behandlung mittels Stromstößen oder durch mechanical bonding erfolgen, wozu Wasserstrahlverfestigung (Spunlace oder Hydro-Entanglement) oder Vernadelung (Needle punching) gehören.

Die Reinigungsschicht eines erfindungsgemäßen Einweg-Tuchprodukts umfasst ein oder besteht vorzugsweise aus einem Mesh-Material, das Pulp (Cellulose-Brei) und Lyocellfasern umfasst oder aus diesen besteht. In dem Mesh-Material, das vorzugsweise ein Flächengewicht von 50-60 g/m² aufweist, sind die Fasern gitterartig angeordnet, was der Reinigungsschicht eine gewisse Steifigkeit und Abrasivität verleiht. In einer alternativen Ausführungsform kann die Reinigungsschicht jedoch auch Viskosefasern in Kombination mit Hanffasern und/oder Flachsfasern enthalten oder daraus bestehen. Hanf- und Flachsfasern weisen von Natur aus bereits eine recht hohe Steifigkeit auf, sodass in diesem Fall keine Mesh-Anordnung erforderlich ist.

Die Kernschicht eines erfindungsgemäßen Einweg-Tuchprodukts enthält vorzugsweise zu mindestens 50 Gew.-%, zu mindestens 60 Gew.-%, zu mindestens 70 Gew.-%, zu mindestens 80 Gew.-%, zu mindestens 90 Gew.-%, zu mindestens 95 Gew.-% oder zu 100 Gew.-% cellulose-basierte Fasern. Vorzugsweise handelt es sich bei den in der Kernschicht enthaltenen cellulose-basierten Fasern um DRC (double re-crepe)-Cellulose, insbesondere mit einem Flächengewicht von 115 g/m².

Cellulosefasern zeichnen sich durch eine hervorragende Speicherkapazität für Tränkungsmittel aus. Daher dient die Kernschicht eines erfindungsgemäßen imprägnierten Einweg-Tuchprodukts als Flüssigkeitsreservoir für das Tränkungsmittel.

DRC-Cellulose im Besonderen zeichnet sich zusätzlich zu der hervorragenden Speicherkapazität für Flüssigkeiten auch durch eine außerordentliche Stabilität im nassen Zustand aus.

Der besondere Vorteil von DRC-Cellulose gegenüber Airlaid-Cellulose liegt darin, dass DRC-Cellulose bei nur etwa ¼ der Dicke von Airlaid-Cellulose deutlich widerstandsfähiger ist, insbesondere um den Faktor 2-3.

Alternativ kann die Kernschicht auch aus 100 % Viskose (Nonwoven-Material) bestehen, insbesondere mit einem Flächengewicht von ca. 100 g/m². Als weitere Alternative kann die Kernschicht auch aus bindemittelfreier oder mit bioabbaubaren Bindemitteln versetzter Airlaid-Cellulose, insbesondere mit einem Flächengewicht von 135 g/m² bestehen. Als weitere Alternative kann die Kernschicht auch aus gepresster Cellulose, insbesondere mit einem Flächengewicht von 160 g/m² bestehen.

Erfindungsgemäß weist das Einweg-Tuchprodukt keinen abrasiven Kratzstreifen auf, wie dieser von dem in der DE 10 2021 122 041 B1 offenbarten Bodenwischtuch bekannt ist. Denn ein solcher abrasiver Kratzstreifen ist zwar für die Bodenreinigung äußerst nützlich, aber da das erfindungsgemäße Einweg-Tuchprodukt für die Körperreinigung und/oder -pflege und/oder für die Reinigung und/oder Pflege von medizinischen Sonden, elektronischen Geräten, Fenstern, Möbeln und/oder Fahrzeugen geeignet sein soll, wäre ein abrasiver Kratzstreifen für die vorgenannten Zwecke, bei denen jeweils kratzempfindliche Oberflächen mit dem Tuchprodukt zu behandeln sind, nicht geeignet.

Das erfindungsgemäße imprägnierte Einweg-Tuchprodukt weist insbesondere ein die Reinigungsschicht, die Kernschicht und optionale weitere Schichten einschließendes Flächengewicht von < 100 g/m² auf, insbesondere ein Flächengewicht (im nicht imprägnierten Zustand) von 15 bis 99 g/m². Hier liegt ein weiterer Unterschied zu dem Bodenwischtuch der DE 10 2021 122 041 B1, denn Bodenwischtücher weisen im nicht imprägnierten Zustand deutlich höhere Flächengewichte von in der Regel 100 bis 250 g/m² auf.

Das Tränkungsmittel, mit dem das erfindungsgemäße Einweg-Tuchprodukt imprägniert ist, enthält vorzugsweise zumindest eine Verbindung mit antimikrobiellen Eigenschaften. Weiterhin kann das Tränkungsmittel sämtliche in herkömmlichen Tränkungsmitteln enthaltene Inhaltsstoffe enthalten, insbesondere Wasser, Öl, Konservierungsmittel, Tenside, Emulgatoren, hautpflegende Inhaltsstoffe, feuchtigkeitsspendende Inhaltsstoffe und beliebige Kombinationen dieser.

Die Bezeichnung "imprägniert" wird im Rahmen der vorliegenden Erfindung gleichbedeutend mit "getränkt" verwendet und meint, dass zumindest eine Schicht der Kernschicht und der Reinigungsschicht mit einem Tränkungsmittel versehen wurde. Die Bezeichnung "imprägniert" schließt dabei auch eine Tränkung mit anschließender Trocknung ein.

Gemäß einer bevorzugten Ausführungsform weist das erfindungsgemäße mehrlagige Einweg-Tuchprodukt die folgenden Schichten und Zusammensetzungen auf:

| | |
|---|---|
| Deckschicht: | 100 % Viskose |
| Reinigungsschicht: | 70 % Hanf / 30 % Lyocell |
| Kernschicht: | 100 % Cellulose |

Gemäß einer weiteren bevorzugten Ausführungsform weist das erfindungsgemäße mehrlagige Einweg-Tuchprodukt die folgenden Schichten und Zusammensetzungen auf:

| | |
|---|---|
| Deckschicht: | 100 % Viskose |
| Reinigungsschicht: | 30 % Hanf / 70 % Viskose |
| Kernschicht: | 100 % Cellulose |

Gemäß einer weiteren bevorzugten Ausführungsform weist das erfindungsgemäße mehrlagige Einweg-Tuchprodukt die folgenden Schichten und Zusammensetzungen auf:

| | |
|---|---|
| Deckschicht: | 100 % Viskose |
| Reinigungsschicht: | 70 % Hanf / 30 % Viskose |
| Kernschicht: | 100 % Cellulose |

Gemäß einer weiteren bevorzugten Ausführungsform weist das erfindungsgemäße mehrlagige Einweg-Tuchprodukt die folgenden Schichten und Zusammensetzungen auf:

| | |
|---|---|
| Deckschicht: | 100 % Viskose |
| Reinigungsschicht: | 100 % Mesh-Material (Pulp/Lyocell) |
| Kernschicht: | 100 % Cellulose |

Gemäß einer weiteren bevorzugten Ausführungsform weist das erfindungsgemäße mehrlagige Einweg-Tuchprodukt die folgenden Schichten und Zusammensetzungen auf:

| | |
|---|---|
| Deckschicht: | 100 % Viskose |
| Reinigungsschicht: | 50 % Hanf / 50 % Lyocell |
| Kernschicht: | 100 % Cellulose |

Die Erfindung betrifft auch ein Verfahren zur Herstellung eines mehrlagigen Einweg-Tuchprodukts, umfassend die Schritte
- Bereitstellen von einer Reinigungsschicht und einer Kernschicht, wobei sowohl die Reinigungsschicht als auch die Kernschicht cellulose-basierte Fasern enthalten und vollständig aus Naturfasern und/oder Regeneratfasern und/oder einem oder mehreren Biopolymer(en) bestehen;
- Verbinden der Reinigungsschicht mit der Kernschicht, um ein Tuchprodukt zu erhalten;
- Imprägnieren des erhaltenen Tuchprodukts mit einem Tränkungsmittel um ein imprägniertes Tuchprodukt zu erhalten.

Das in dem erfindungsgemäßen Verfahren hergestellte Einweg-Tuchprodukt weist die Eigenschaften auf, die im Voranstehenden mit Bezug auf das erfindungsgemäße Einweg-Tuchprodukt als solches offenbart wurden, weshalb die vorangegangene Beschreibung auch zur näheren Charakterisierung des Verfahrens herangezogen werden kann. Dies bedeutet insbesondere, dass
- die in dem erfindungsgemäßen Verfahren bereitgestellte Reinigungsschicht sowie die Kernschicht dieselbe bevorzugte Zusammensetzung aufweisen können wie die in dem erfindungsgemäßen Einweg-Tuchprodukt enthaltene Reinigungsschicht und Kernschicht; und/oder
- zusätzlich zu der Kernschicht und der Reinigungsschicht noch eine optionale Deckschicht in dem erfindungsgemäßen Verfahren bereitgestellt wird, wobei die Deckschicht ebenfalls vollständig aus Naturfasern und/oder Regeneratfasern und/oder einem oder mehreren Biopolymeren besteht und auf der der Reinigungsschicht abgewandten Seite der Kernschicht angeordnet wird; und/oder
- zusätzlich zu der Kernschicht und der Reinigungsschicht noch eine optionale flüssigkeitsundurchlässige Sperrschicht in dem erfindungsgemäßen Verfahren bereitgestellt wird, die vorzugsweise aus nachwachsenden und/oder biologisch abbaubaren Rohstoffen besteht und auf der der Reinigungsschicht abgewandten Seite der Kernschicht angeordnet wird.

Vorzugsweise erfolgt in dem erfindungsgemäßen Verfahren das Verbinden der Reinigungsschicht und der Kernschicht sowie optionaler weiterer Schichten, insbesondere der vorgenannten Deckschicht und/oder der Sperrschicht, mittels Mikro-Entangling durch mittels Ultraschall erzeugter Bewegung, durch eine Behandlung mittels Stromstößen, durch Wasserstrahlverfestigung und/oder durch Needlepunching.

Die Erfindung betrifft weiterhin auch die Verwendung eines mehrlagigen Einweg-Tuchprodukts für die Körperreinigung und/oder -pflege sowie in einer weiteren Ausführungsform auch die Verwendung für die Reinigung und/oder Pflege von medizinischen Sonden, elektronischen Geräten, Fenstern, Möbeln und/oder Fahrzeugen.

Das in den erfindungsgemäßen Verwendungen verwendete Einweg-Tuchprodukt weist die Eigenschaften auf, die im Voranstehenden mit Bezug auf das erfindungsgemäße Einweg-Tuchprodukt als solches offenbart wurden, weshalb die vorangegangene Beschreibung des erfindungsgemäßen Einweg-Tuchprodukts auch zur näheren Charakterisierung der erfindungsgemäßen Verwendung herangezogen werden kann.

Die in der vorstehenden Beschreibung, in den Beispielen sowie in den Ansprüchen offenbarten Merkmale der Erfindung können sowohl einzeln als auch in beliebigen Kombinationen für die Verwirklichung der Erfindung in ihren verschiedenen Ausführungsformen wesentlich sein.

## Patentansprüche

1. Mehrlagiges imprägniertes Einweg-Tuchprodukt für die Körperreinigung und/oder -pflege und/oder für die Reinigung und/oder Pflege von medizinischen Sonden, elektronischen Geräten, Fenstern, Möbeln und/oder Fahrzeugen, wobei das Einweg-Tuchprodukt mindestens eine Reinigungsschicht und eine Kernschicht umfasst, **dadurch gekennzeichnet, dass** sowohl die Reinigungsschicht als auch die Kernschicht cellulose-basierte Fasern enthalten und vollständig aus Naturfasern und/oder Regeneratfasern und/oder einem oder mehreren Biopolymeren bestehen.

2. Mehrlagiges Einweg-Tuchprodukt nach Anspruch 1, **gekennzeichnet durch** eine Deckschicht, die ebenfalls vollständig aus Naturfasern und/oder Regeneratfasern und/oder einem oder mehreren Biopolymeren besteht und auf der der Reinigungsschicht abgewandten Seite der Kernschicht angeordnet ist.

3. Mehrlagiges Einweg-Tuchprodukt nach Anspruch 1 oder 2, **dadurch gekennzeichnet, dass** die in der Reinigungsschicht, in der Kernschicht und in der optionalen Deckschicht enthaltenen Naturfasern und/oder Regeneratfasern unabhängig voneinander aus der Gruppe ausgewählt sind, die Viskosefasern, Modalfasern, Lyocellfasern, Cellulosefasern, Cuprofasern, Baumwollfasern, Jutefasern, Hanffasern und Flachsfasern sowie beliebige Mischungen dieser umfasst oder daraus besteht.

4. Mehrlagiges Einweg-Tuchprodukt nach einem der voranstehenden Ansprüche, **dadurch gekennzeichnet, dass** die Reinigungsschicht aus einem Mesh-Material besteht, das Pulp und Lyocellfasern umfasst oder aus diesen besteht, oder Viskosefasern in Kombination mit Hanffasern und/oder Flachsfasern umfasst oder daraus besteht.

5. Mehrlagiges Einweg-Tuchprodukt nach einem der voranstehenden Ansprüche, **dadurch gekennzeichnet, dass** die Kernschicht zu 100 % aus cellulose-basiertem Material besteht.

6. Mehrlagiges Einweg-Tuchprodukt nach einem der voranstehenden Ansprüche, **dadurch gekennzeichnet, dass** dieses aus der Reinigungsschicht, der Kernschicht und optional einer oder mehreren weiteren Schichten besteht, wobei die eine oder die mehreren weiteren Schichten vollständig aus Naturfasern und/oder Regeneratfasern und/oder einem oder mehreren Biopolymeren bestehen.

7. Mehrlagiges Einweg-Tuchprodukt nach einem der voranstehenden Ansprüche, **dadurch gekennzeichnet, dass** dieses im nicht imprägnierten Zustand ein die Reinigungsschicht, die Kernschicht und optionale weitere Schichten einschließendes Flächengewicht von < 100 g/m² aufweist, insbesondere ein Flächengewicht von 15 bis 99 g/m².

8. Mehrlagiges Einweg-Tuchprodukt nach einem der voranstehenden Ansprüche, **dadurch gekennzeichnet, dass** dieses im imprägnierten Zustand 80-1.200 Gew.-% Tränkungsmittel umfasst, bezogen auf das Gewicht des mehrlagigen Einweg-Tuchprodukts im nicht imprägnierten Zustand.

9. Mehrlagiges Einweg-Tuchprodukt nach einem der voranstehenden Ansprüche, **dadurch gekennzeichnet, dass** das Tuchprodukt ausgewählt ist aus der Gruppe, die feuchte Waschlappen, feuchte Waschhandschuhe, mit einem Tränkungsmittel getränkte Gesichtsreinigungstücher, feuchtes Toilettenpapier, mit einem Tränkungsmittel getränkte Babytücher und mit einem Tränkungsmittel getränkte Reinigungstücher umfasst.

10. Verfahren zur Herstellung eines mehrlagigen imprägnierten Einweg-Tuchprodukts für die Körperreinigung und/oder -pflege und/oder für die Reinigung und/oder Pflege von medizinischen Sonden, elektronischen Geräten, Fenstern, Möbeln und/oder Fahrzeugen nach einem der voranstehenden Ansprüche, umfassend die Schritte
- Bereitstellen von einer Reinigungsschicht und einer Kernschicht sowie optional einer oder mehrerer weiterer Schichten, wobei sowohl die Reinigungsschicht als auch die Kernschicht und die eine oder mehreren optionalen weiteren Schichten cellulose-basierte Fasern enthalten und vollständig aus Naturfasern und/oder Regeneratfasern und/oder einem oder mehreren Biopolymeren bestehen;
- Verbinden der Reinigungsschicht mit der Kernschicht sowie optionaler weiterer Schichten, um ein Tuchprodukt zu erhalten;
- Imprägnieren des erhaltenen Tuchprodukts mit einem Tränkungsmittel.

11. Verfahren nach Anspruch 10, **dadurch gekennzeichnet, dass** die Reinigungsschicht und die Kernschicht sowie eine oder mehrere optionale weitere Schichten miteinander durch eine Behandlung mittels Stromstößen, durch Wasserstrahlverfestigung, durch Needlepunching und/oder mittels Mikro-Entangling über durch Ultraschall erzeugte Bewegungen der in den jeweiligen Schichten enthaltenen Fasern verbunden werden.

12. Verwendung eines mehrlagigen imprägnierten Einweg-Tuchprodukts nach einem der Ansprüche 1 bis 9 für die Körperreinigung und/oder -pflege.

13. Verwendung nach Anspruch 12, **dadurch gekennzeichnet, dass** das imprägnierte Einweg-Tuchprodukt ein feuchter Waschlappen, ein feuchter Waschhandschuh, ein mit einem Tränkungsmittel getränktes Gesichtsreinigungstuch, feuchtes Toilettenpapier, ein mit einem Tränkungsmittel getränktes Babytuch oder ein mit einem Tränkungsmittel getränktes Reinigungstuch ist.

14. Verwendung eines mehrlagigen imprägnierten Einweg-Tuchprodukts nach einem der Ansprüche 1 bis 9 für die Reinigung und/oder Pflege von medizinischen Sonden, elektronischen Geräten, Fenstern, Möbeln und/oder Fahrzeugen.
